# EUROPÄISCHE PATENTSCHRIFT

(11) **EP 1 104 406 B1**
(45) Veröffentlichungstag und Bekanntmachung des Hinweises auf die Patenterteilung: **23.11.2005**
(21) Anmeldenummer: 99940140.9
(22) Anmeldetag: 04.08.1999
(51) Int. Cl.: C07D 231/14

(54) **VERFAHREN ZUR HERSTELLUNG VON 1-ALKYL-PYRAZOL-5-CARBONSÄUREESTERN III**
METHOD FOR PREPARING 1-ALKYL-PYRAZOL-5-CARBOXYLIC ACID ESTERS III
PROCEDE DE PREPARATION D'ESTERS III D'ACIDE 1-ALKYLE-PYRAZOLE-5-CARBOXYLIQUE

(30) Priorität: 17.08.1998 DE 19837067
(43) Veröffentlichungstag der Anmeldung: 06.06.2001
(73) Patentinhaber: LANXESS Deutschland GmbH, 51369 Leverkusen (DE)
(72) Erfinder: MÜLLER, Nikolaus, D-40789 Monheim (DE); MATZKE, Michael, D-42113 Wuppertal (DE)
(86) Internationale Anmeldenummer: PCT/EP1999/005641
(87) Internationale Veröffentlichungsnummer: WO 2000/010978

(56) Entgegenhaltungen:
- EP-A- 0 029 363
- EP-A- 0 854 142

## Beschreibung

Die vorliegende Erfindung betrifft ein besonders vorteilhaftes Verfahren zur Herstellung von 1-Alkyl-, insbesondere 1,3-Dialkyl-pyrazol-5-carbonsäureestern, aus 2,4-Diketosäureestem und Alkylhydrazinen, wobei die 2,4-Diketosäureester ganz oder teilweise in Form ihrer Enolate und die Alkylhydrazine teilweise in Form von Alkylhydraziniumsalzen eingesetzt werden könnnen.

Es ist bekannt, 1-Alkyl-pyrazol-5-carbonsäureester herzustellen, indem man 2,4-Diketocarbonsäureester und Alkylhydrazine miteinander umsetzt. Dabei werden Isomerengemische erhalten, die in der Regel überwiegend das hier unerwünschte Isomere enthalten, was ein aufwendiges Trennverfahren nötig macht und die Ausbeute an gewünschtem Isomeren niedrig hält. So ergibt die Umsetzung von vorgelegtem 2,4-Dioxo-pentancarbonsäureethylester mit Methylhydrazin ein 1:1-Gemisch aus 1,5-Dimethyl-pyrazol-3-carbonsäureester und dem entsprechenden 2,5-Dimethylisomeren (Austr. J. Chem. 36, 135-147 (1983)). Andere Autoren berichten für diese Umsetzung noch ungünstigere Verhältnisse von 35:65 (Chem. Ber. 59, 1282 (1926)), die in vergleichenden Laborversuchen bestätigt wurden. Die gleichen Autoren haben mit analogen veretherten Enolen, z.B. mit O-Ethylacetonoxalester und Methylhydrazin, noch schlechtere Ergebnisse erzielt (Isomerenverhältnis 15:85).

Auch die EP-A 029 363 beschreibt die Synthese von N-alkylsubstituierten Pyrazolcarbonsäureestern mit langen Alkylresten aus vorgelegtem Diketoesterenolat und Alkylhydrazin. Das in mäßigen Ausbeuten isolierte Pyrazol ist aber wiederum nicht das erwünschte, da der N-Alkylsubstituent und die Carboxylgruppe in 1,3-Stellung und nicht wie gewünscht in 1,5-Stellung vorliegen. Bei diesen Umsetzungen werden entweder die freien Alkylhydrazine mit vorgelegtem Diketoester umgesetzt oder das Natriumenolat des Diketoesters und ein Alkylhydraziniumsalz vorgelegt und aus dem Hydraziniumsalz das Hydrazin mit basischen Verbindungen (wie Natriumhydroxid oder Soda) in Freiheit gesetzt.

Gemäß der EP-A 854 142 werden 1-Alkyl-pyrazol-5-carbonsäureester hergestellt, indem man das Enolat eines 2,4-Diketocarbonsäureesters in Gegenwart eines Lösungsmittels, z.B. eines Alkohols, mit einem Alkylhydraziniumsalz umsetzt. Dabei ist das Alkylhydraziniumsalz aus Alkylhydrazin mit einer Säure in Gegenwart von Alkohol herzustellen. Die Isomerenverhältnisse liegen hier zwar günstig, die Menge an unerwünschtem Isomer ist jedoch nicht unbeträchtlich und deshalb die Ausbeute noch nicht optimal.

Es besteht deshalb noch das Bedürfnis nach einem Verfahren zur selektiven Herstellung von 1-Alkyl-pyrazol-5-carbonsäureestern, bei dem deutlich weniger des entsprechenden 1,3-Isomeren gebildet werden, als bei den bekannten Verfahren.

Es wurde nun ein Verfahren zur Herstellung von 1-Alkyl-pyrazol-5-carbonsäure-estern der Formel (I) gefunden 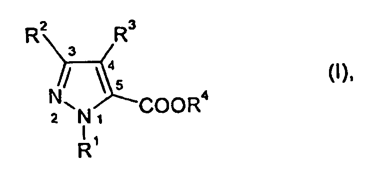 in der
- R¹ und R⁴: unabhängig voneinander jeweils für geradkettiges oder verzweigtes, gegebenenfalls halogensubstituiertes C₁-C₆-Alkyl, C₃-C₇-Cycloalkyl oder gegebenenfalls substituiertes C₇-C₁₂Aralkyl und
- R² und R³: unabhängig voneinander jeweils für Wasserstoff, geradkettiges oder verzweigtes, gegebenenfalls durch Halogen substituiertes C₁-C₆-Alkyl, gegebenenfalls durch Halogen substituiertes C₃-C₇-Cycloalkyl oder gegebenenfalls substituiertes C₇-C₁₂-Aralkyl stehen,
das dadurch gekennzeichnet ist, daß man einen 2,4-Diketoester der Formel 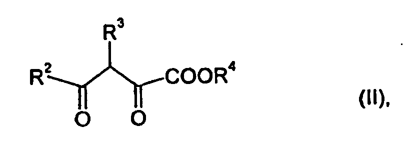 in der
R², R³ und R⁴ die bei Formel (I) angegebene Bedeutung haben,
und/oder ein Enolat davon
zu einem Alkylhydrazin der Formel

R¹-NH-NH (III),

in der
- R¹: die bei Formel (I) angegebene Bedeutung hat,
und/oder einem entsprechenden Alkylhydraziniumsalz,
gegebenenfalls in Gegenwart von einem Lösungsmittel und/oder von Wasser gibt und weiterhin so viel freies Alkylhydrazin der Formel (III) und so viel Alkylhydraziniumsalz einsetzt, daß nach Beendigung der Umsetzung noch 5 bis 200 Mol-% freies Alkylhydrazin der Formel (III) vorliegen (bezogen auf eingesetzten Diketoester bzw. sein Enolat).

C₇-C₁₂-Aralkyl und das daraus bevorzugte Benzyl sowie C₆-C₁₀-Aryl (später genannt) und das daraus bevorzugte Phenyl (später genannt) können beispielsweise jeweils bis zu zwei Substituenten aus der Gruppe der Halogenatome und der C₁-C₄-Alkylreste enthalten.

Als Diketoester der Formel (II) sind solche bevorzugt, bei denen die Reste R² und R³ unabhängig voneinander jeweils für Wasserstoff, geradkettiges oder verzweigtes C₁-C₄-Alkyl, C₃-C₆-Cycloalkyl oder gegebenenfalls substituiertes Benzyl stehen sowie solche, bei denen der Rest R⁴ für geradkettiges oder verzweigtes C₁-C₄-Alkyl steht.

Besonders bevorzugt sind 2,4-Diketoester der Formel (II), bei denen R² und R⁴ jeweils für C₁-C₄-Alkyl und R³ für H stehen.

Von den Alkylhydrazinen der Formel (III) sind solche bevorzugt, bei denen R¹ für geradkettiges oder verzweigtes C₁-C₄-Alkyl oder gegebenenfalls substituiertes Benzyl steht.

Diketoester der Formel (II) können nach einer üblichen Methode durch Kondensation eines Dialkylketons der Formel 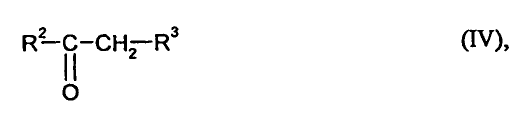 in der
R² und R³ die bei Formel (I) angegebenen Bedeutungen haben,
mit einem Oxalester der Formel

R⁴OOC-COOR⁴ (V),

in der
- R⁴: die bei Formel (I) angegebene Bedeutung hat,
hergestellt werden. Man arbeitet hierbei in Gegenwart von basischen Kondensationsmitteln, z.B. Alkoholaten, und eines Lösungsmittels. Aus der dabei erhaltenen rohen Reaktionsmischung kann der Diketoester der Formel (II), der als Enolat anfällt, durch Ansäuern freigesetzt und auf üblichem Wege, z.B. durch Extraktion mit einem organischen Lösungsmittel, Einengen und Destillation in reiner Form gewonnen werden (siehe Organikum, 16. Auflage, 1976, S. 472).

Als Lösungsmittel für die Umsetzung der Dialkylketone der Formel (IV) mit Oxalestern der Formel (V) kommen beispielsweise Alkohole wie Methanol, Ethanol, n-Propanol, i-Propanol und n-, i-, s- und t-Butanol in Frage. Das Alkoholat kann durch Auflösen eines Alkali- oder Erdalkalimetalls in dem Alkohol hergestellt werden, der dem Alkoholat entspricht.

Der Diketoester der Formel (II) wird erfindungsgemäß mit dem Alkylhydrazin der Formel (III) so umgesetzt, daß während mindestens 90 % der Umsetzung freies Alkylhydrazin der Formel (III) vorliegt. Vorzugsweise liegt während 95 bis 100 % der Umsetzung freies Alkylhydrazin vor. Unter "x % der Umsetzung" wird dabei verstanden, daß x % des insgesamt eingesetzten Diketoesters der Formel (II) umgesetzt worden sind.

Dies kann am einfachsten erreicht werden, indem man das Alkylhydrazin der Formel (III) in äquimolarer Menge oder im Überschuß, gegebenenfalls in einem Lösungsmittel oder Wasser, vorlegt und den Diketoester der Formel (II), gegebenenfalls in einem Lösungsmittel, langsam zugibt. Durch das Vorlegen des Alkylhydrazins der Formel (III) wird gewährleistet, daß selbst beim Einsatz stöchiometrischer Mengen von Alkylhydrazin und Diketoestern während nahezu 100 % der Umsetzung freies Alkylhydrazin der Formel (III) vorliegt.

Man kann auch eine kleine Menge freies Alkylhydrazin vorlegen, z.B. 10 bis 20 Gew.-% der Gesamtmenge, und den Rest des Alkylhydrazins und den Diketoester, beide gegebenenfalls in einem Lösungsmittel oder Wasser, simultan zugeben. Es ist dabei vorteilhaft, das Alkylhydrazin mit einem kleinen Vorlauf zuzugeben.

Bevorzugt setzt man den Diketoester der Formel (II) in Form eines Enolates der Formel (VI) ein: 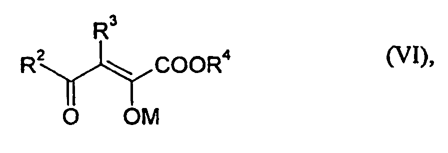 in der
R², R³ und R⁴ die bei Formel (I) angegebene Bedeutung haben und
- M: für ein Äquivalent eines Alkali- oder Erdalkalimetalls steht.

Wenn man Alkylhydraziniumsalze einsetzt, können diese der Formel (VII) entsprechen

[R¹NH₂-NH₂]⁺ X⁻ (VII),

in der
- R¹: die bei Formel (I) angegebene Bedeutung hat und
- X⁻: für das Anion einer organischen oder anorganischen Säure steht.

Als Anion X- von anorganischen Säuren kommen beispielsweise Chlorid, Bromid, Fluorid, Hydrogensulfat, Dihydrogenphosphat und Hydrogencarbonat in Betracht. Als Anionen X- sind solche von organischen Säuren bevorzugt. Insbesondere steht dann X- für R⁵COO-, wobei R⁵ einen C₁-C₁₀-aliphatischen oder einen C₆-C₁₂-aromatischen Rest bedeutet, die jeweils gegebenenfalls substituiert sein können. Bevorzugt steht R⁵ für Formiat, Acetat, Propionat, Butyrat oder Benzoat.

R⁵ kann zusammen mit dem COO--Teil auch für ein Anion einer mehrbasischen organischen Säure stehen. Beispiele sind Anionen von Oxal-, Malon-, Bernstein-, Glutar-, Adipin-, Malein-, Fumar-, Äpfel-, Wein- und Zitronensäure, wobei es sich jeweils um Mono- oder Polyanionen handeln kann. Solche Anionen können einen oder mehrere COO--Teile und gegebenenfalls zusätzlich auch COOH-Reste enthalten.

Im Falle von Anionen von mehrbasischen organischen Säuren kann Alkylhydrazin im Verhältnis zur Säure in äquivalenten Mengen, also in gleicher Anzahl wie die Anzahl Säuregruppen, vorliegen.

Um auch beim Einsatz von Alkylhydraziniumsalzen das erfindungsgemäß erforderliche freie Alkylhydrazin der Formel (III) vorliegen zu haben, wird dem Alkylhydraziniumsalz zweckmäßigerweise vor Beginn der Reaktion mit dem Diketoester oder dessem Enolat freies Alkylhydrazin der Formel (III) zugesetzt.

Man kann das erfindungsgemäße Verfahren beispielsweise durchführen, indem man ein Diketoesterenolat der Formel (VI), beispielsweise in Form einer rohen Reaktionsmischung der Kondensationsreaktion zwischen einem Keton der Formel (IV) und einem Oxalester der Formel (V), zu einem Gemisch aus einer möglichst äquimolaren Menge eines Alkylhydraziniumsalzes der Formel (VII) zudosiert, das einen Anteil an freiem Alkylhydrazin der Formel (III) enthält. Aus dem Diketoesterenolat der Formel (VI) wird dabei durch Reaktion mit dem Alkylhydraziniumsalz der Formel (VII) der freie Diketoester der Formel (II) und freies Alkylhydrazin der Formel (III) in situ gebildet, die spontan zum gewünschten Pyrazol der Formel (I) reagieren.

Ein Alkylhydraziniumsalz der Formel (VII) im Gemisch mit freiem Alkylhydrazin der Formel (III), gegebenenfalls in Gegenwart eines Lösungsmittels oder Wasser, kann aber auch zu vorgelegtem Diketoester der Formel (II) oder einem Enolat davon der Formel (VI) gegeben werden. So wird gewährleistet, daß aus dem Enolat freigesetzter Diketoester immer auf freies Alkylhydrazin trifft.

Werden Lösungsmittel und/oder Wasser angewendet, so werden die insgesamt eingesetzten Lösungsmittel- und Wassermengen im allgemeinen so gewählt, daß rührbare Suspensionen oder Lösungen vorliegen. Die Gesamtmenge Lösungsmittel plus Wasser pro Mol Reaktionsansatz kann z.B. zwischen 100 und 2 000 ml liegen. Bevorzugt beträgt diese Menge 200 bis 1000 ml, besonders bevorzugt 250 bis 500 ml. Von der Gesamtmenge Lösungsmittel plus Wasser können beispielsweise 10 bis 60 Gew.-%, vorzugsweise 15 bis 40 Gew.-%; Wasser sein.

Es ist vorteilhaft, in das erfindungsgemäße Verfahren so viel freies Alkylhydrazin der Formel (III) und gegebenenfalls so viel Alkylhydraziniumsalz der Formel (VII) einzusetzen, daß nach Beendigung der Umsetzung beispielsweise noch 5 bis 200 Mol-% freies Alkylhydrazin der Formel (III) vorliegen (bezogen auf eingesetzten Diketoester bzw. sein Enolat). Besonders bevorzugt liegt diese Menge bei 5 bis 50 Mol%, insbesondere bei 5 bis 20 Mol-%.

Wenn man die Herstellung des Diketoesterenolats der Formel (VI) in Gegenwart von Alkoholat durchführt, kann die Alkoholatmenge in weiten Grenzen schwanken. Vorzugsweise werden mindestens 90 Mol-% Alkoholat, bezogen auf das Keton der Formel (IV) eingesetzt.

Wird der Diketoester der Formel (II) in einem vorgeschalteten Schritt wie oben beschrieben aus einem Dialkylketon der Formel (IV) und einem Oxalsäureester der Formel (V) hergestellt, so können auch deren Molverhältnisse variieren. Bevorzugt arbeitet man mit einem leichten Überschuß an Oxalsäureester, beispielsweise mit einem Unterschuß von je 1 bis 10 Mol-% Dialkylketon der Formel (IV) und Alkoholat der Formel

M(OR⁶)ₙ (VIII),

in der
- M: die bei Formel (VI) angegebene Bedeutung hat,
- R⁶: für C₁-C₄-Alkyl steht und
- n: der Wertigkeit von M entspricht.

Das Dialkylketon und das Alkoholat werden bevorzugt in etwa äquimolaren Mengen zueinander eingesetzt. Insgesamt kann man beispielsweise auf 0,9 bis 0,99 Mol Dialkylketon der Formel (IV) 0,9 bis 1,1 Mol Oxalsäureester der Formel (V) und 0,9 bis 1,1 Mol Alkoholat, beispielsweise der Formel (VIII), einsetzen.

Stellt man Alkylhydraziniumsalz der Formel (VII) aus einem Alkylhydrazin der Formel (III) und einer Carbonsäure beispielsweise der Formel R⁵COOH her, so ist es zweckmäßig, daß Molverhältnis der beiden Einsatzstoffe so zu wählen, daß nach Bildung des Alkylhydraziniumsalzes darin beispielsweise 5 bis 200 Mol-% freies Alkylhydrazin der Formel (III) vorliegen.

Ein Überschuß an Carbonsäure, beispielsweise der Formel R⁵COOH ist nur dann vorteilhaft, wenn Alkoholat, z.B. aus der Herstellung eines Diketoesterenolats der Formel (VI), im Überschuß vorliegt. Dann kann der Alkoholatüberschuß mit dem Säureüberschuß neutralisiert werden.

Die Reaktionstemperaturen für die erfindungsgemäße Umsetzung können z.B. zwischen -20 und +100°C liegen. Bevorzugt sind 0 bis 80°C, besonders bevorzugt 0 bis 50°C. Solche Temperaturen können auch während einer eventuellen Nachrührzeit eingehalten werden.

Die Reaktionszeit (= Zeit für das Zusammengeben der Reaktionspartner + Nachrührzeit) kann z.B. zwischen 0,5 und 12 Stunden betragen. Bevorzugt liegen sie zwischen 1 und 8 Stunden, besonders bevorzugt zwischen 2 und 5 Stunden.

In das erfindungsgemäße Verfahren werden bevorzugt folgende 2,4-Diketoester-Komponenten eingesetzt: 2,4-Diketopentancarbonsäureethylester als Natrium-, Lithium-, Kalium- oder Magnesiumenolatsalze, 2,4-Diketohexancarbonsäureethylester, 2,4-Diketoheptancarbonsäureethylester, 2,4-Diketooctancarbonsäureethylester und 2,4-Diketo-3-ethylpentancarbonsäureethylester (jeweils in Form ihrer Natrium-, Lithium-, Kalium- oder Magnesiumenolatsalze) oder Methyl-, n-Propyl-, i-Propyl- und n-, i-, s- und t-Butylester der zuvor genannten Diketocarbonsäuren in Form der genannten Enolatsalze.

Bevorzugte Alkylhydrazine der Formel (III) sind Methyl-, Ethyl-, n-Propyl-, i-Propyl-, n-Butyl-, t-Butyl-, Benzyl-und n-Pentylhydrazin.

Eine allgemeine Ausführungsform des erfindungsgemäßen Verfahrens wird im folgenden beispielhaft an der Umsetzung von 2,4-Diketoheptancarbonsäureethylester mit Methylhydrazin erläutert:

Aus 2-Pentanon und Oxalsäurediethylester wird zunächst mit Natriumethylat als Hilfsbase in Ethanol das Natriumsalz des 2,4-Diketoheptancarbonsäureethylesters analog bekannter Vorschriften hergestellt (siehe z.B. Organikum, 19. Aufl., S. 490 (1993)). Diese Lösung wird, um ein Ausfallen des Enolats zu verhindern, auf 50°C gehalten und innerhalb von 1 Stunde zu einer zuvor hergestellten Mischung aus Methylhydrazin und Essigsäure (Molverhältnis 1,1:1) gegeben. Man rührt nach, destilliert das überschüssige Ethanol ab, versetzt mit Toluol und gegebenenfalls mit weiterem Wasser. Zur Verbesserung der Trennung der Phasen kann man gegebenenfalls ein geeignetes Tensid, z.B. ein Alkansulfonat, zufügen und/oder die Ionenkonzentration in der wäßrigen Phase erhöhen, z.B. durch Zusatz eines Salzes, etwa eines Alkalihalogenids. Die Toluolphase wird dann abgetrennt und die wäßrige Phase noch 2 mal mit Toluol extrahiert. Nach Vereinigung der organischen Phasen werden diese mit Wasser reextrahiert. Das Wasser kann gegebenenfalls eine Säure und/oder ein Salz enthalten. Die toluolische Lösung des Rohpyrazols wird schließlich durch Destillation des Lösungsmittels eingeengt und der Rückstand im Vakuum fraktioniert destilliert. Die beiden isomeren Pyrazole lassen sich ohne großen Trennaufwand in reiner Form isolieren.

Mit dem erfindungsgemäßen Verfahren erhält man die gewünschten 1-Alkyl-pyrazol-5-carbonsäureester im allgemeinen in über 8-facher Menge, häufig in 10-fächer oder noch höherer Menge, jeweils bezogen auf das unerwünschte Fehlisomere (= 1-Alkyl-pyrazol-3-carbonsäureester).

Es ist ausgesprochen überraschend, daß das erfindungsgemäße Verfahren die Regioselektivität der Bildung der gewünschten 1-Alkyl-pyrazol-5-carbonsäureester so außerordentlich günstig beeinflußt. Wenn man während der Umsetzung kein freies Alkylhydrazin der Formel (III) vorliegen hat, wie das gemäß dem Stand der Technik gemacht wird (siehe auch Vergleichsbeispiel) erhält man die unerwünschten Isomeren in größerer Menge als die gewünschten Isomeren der Formel (I) oder das gewünschte Isomere in deutlich geringerem Überschuß. Besonders überraschend ist zu vermerken, daß schon ein kleiner Überschuß an freiem Alkylhydrazin die aufgezeigte weitgehende Umkehr der Isomerenverteilung bewirkt.

1-Alkyl-pyrazol-5-carbonsäureester der Formel (I) sind wertvolle Zwischenprodukte zur Herstellung von pharmazeutischen Wirkstoffen mit gefäßverändernder und/oder krampflösender Wirkung (siehe EP-OS 463 756, EP-OS 526 004, WO 94/28902 und DE-OS 19 27 429) sowie zur Herstellung von Pestiziden mit insektizider und akarizider Wirkung (siehe JP-OS 89-114 466).

### Beispiele

### Beispiel 1

10,4 g Methylhydrazin wurden in 50 ml Ethanol vorgelegt und unter Rühren und Außenkühlung mit einer Lösung von 42,9 g 2,4-Diketoheptancarbonsäureethylester in 50 ml Ethanol versetzt, wobei die Innentemperatur auf 5 bis 10°C gehalten wurde (Zutropfzeit 10 Minuten). Man rührte 30 Minuten bei 5 bis 10°C nach, destillierte das Lösungsmittel und entstandenes Wasser ab und fraktionierte den Rückstand im Vakuum. Der gewünschte 1-Methyl-3-n-propyl-pyrazol-5-carbonsäureethylester ging bei 125 bis 128°C (13 mm) über. Die Ausbeute betrug 78,3 % d.Th. Durch weitere Destillation (166 bis 168°C/13 mm) wurde das unerwünschte Isomere (= 1-Methyl-5-n-propyl-pyrazol-3-carbonsäureethylester) in einer Ausbeute von 7,9 % d.Th. erhalten. Das Isomerenverhältnis lag also etwa bei 10:1 zugunsten des gewünschten Isomeren.

### Beispiel 2 (Vergleich)

In Umkehrung der Arbeitsweise gemäß Beispiel 1 wurden 42,9 g 2,4-Diketoheptancarbonsäureethylester in 50 ml Ethanol vorgelegt und 10,4 g Methylhydrazin bei 5 bis 10°C unter Rühren und Außenkühlung während 1 Stunde zugetropft. Es wurden nach der Aufarbeitung und Reindestillation entsprechend der Arbeitsweise gemäß Beispiel 1 40,5 % d.Th. 1-Methyl-3-n-propyl-pyrazol-5-carbonsäureethylester und 51 % d.Th. 1-Methyl-5-n-propyl-pyrazol-3-carbonsäureethylester erhalten. Das Isomerenverhältnis lag also bei etwa 5:4 zugunsten des unerwünschten Isomeren.

### Beispiel 3

In einem 11-Vierhalskolben wurden 1462 g Oxalsäurediethylester vorgelegt und 776 g Pentanon-2 zugegeben. Unter Rühren wurden bei 25 bis 40°C 3400 g einer 20 gew.-%igen Natriumethylatlösung in Ethanol innerhalb einer Stunde zudosiert.

Das Reaktionsgemisch wurde 1 Stunde bei 50°C und anschließend 1 Stunde bei Rückfluß gerührt und nachfolgend wieder auf 50°C abgekühlt. In einem weiteren Kolben wurden während der Nachrührzeit 506 g (10,95 Mol) Methylhydrazin vorgelegt und hierzu 600 g (10 Mol) Essigsäure innerhalb von 1 Stunde bei 5 bis 30°C zugetropft und anschließend auf 10°C gekühlt. Zu diesem Gemisch wurde die zuerst hergestellte, auf 50°C gehaltene 2,4-Diketoheptancarbonsäureethylesterenolat-Lösung innerhalb von 2 Stunden zugetropft, wobei die Temperatur des Reaktionsgemisches zwischen 8 und 15°C und die Temperatur der Esterenolat-Lösung bei 45 bis 55°C gehalten wurde. Nach Beendigung der Zugabe wurden weitere 60 g Eisessig zugegeben und das Lösungsmittel abdestilliert, bis eine Sumpftemperatur von 88°C erreicht war. Der Rückstand wurde abgekühlt und unter Rühren mit 2000 ml Toluol, 5000 ml Wasser, 500 g eines Tensids (Mersolat® H30) und 20 g Natriumchlorid versetzt. Die wäßrige Phase wurde abgetrennt und mit 200 ml Toluol extrahiert. Die organischen Phasen wurden vereinigt, mit 2000 g 5 gew.%iger wäßriger Schwefelsäure und 2000 ml Wasser gewaschen. Anschließend wurde bei 60 mbar eingeengt bis eine Sumpftemperatur von 70°C erreicht war. Es wurden 1748 g eines braunen Öls erhalten, das 74,7 Gew.-% 1-Methyl-3-n-propyl-pyrazol-5-carbonsäure-ethylester und 5,8 Gew.-% unerwünschten 1-Methyl-5-n-propyl-pyrazol-3-carbonsäure-ethylester enthielt, was einer Rohausbeute von 73,9 % d.Th. der gewünschten Verbindung entspricht. Das Isomerenverhältnis lag also bei etwa 13:1 zugunsten des gewünschten Isomeren.

Nach Destillation im Vakuum wurde als 1. Fraktion 1152 g des gewünschten Produkts mit einem Siedepunkt von 121°C bei 11 mbar und einem Gehalt (GC) von 99,9 % erhalten. Die Ausbeute des reinen Produktes betrug 65 % d.Th.

## Patentansprüche

1. Verfahren zur Herstellung von 1-Alkyl-pyrazol-5-carbonsäureestern der Formel 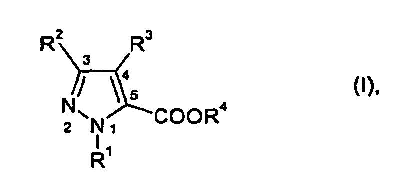 in der
R¹ und R⁴ unabhängig voneinander jeweils für geradkettiges oder verzweigtes, gegebenenfalls durch Halogen substituiertes C₁-C₆-Alkyl, gegebenenfalls durch Halogen substituiertes C₃-C₇-Cycloalkyl oder gegebenenfalls substituiertes C₇-C₁₂-Aralkyl und
R² und R³ unabhängig voneinander jeweils für Wasserstoff, geradkettiges oder verzweigtes, gegebenenfalls halogensubstituiertes C₁-C₆-Alkyl, C₃-C₇-Cycloalkyl oder gegebenenfalls substituiertes C₇-C₁₂-Aralkyl stehen,
**dadurch gekennzeichnet, daß** man einen 2,4-Diketoester der Formel 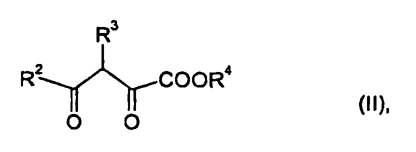 in der
R², R³ und R⁴ die bei Formel (I) angegebene Bedeutung haben,
und/oder ein Enolat davon
zu einem Alkylhydrazin der Formel
R¹-NH-NH₂ (III),
in der
R¹ die bei Formel (I) angegebene Bedeutung hat,
und/oder einem entsprechenden Alkylhydraziniumsalz,
gegebenenfalls in Gegenwart von einem Lösungsmittel und/oder von Wasser gibt, und weiterhin so viel freies Alkylhydrazin der Formel (III) und so viel Alkylhydraziniumsalz einsetzt, daß nach Beendigung der Umsetzung noch 5 bis 200 Mol-% freies Alkylhydrazin der Formel (III) vorliegen (bezogen auf eingesetzten Diketoester bzw. sein Enolat).

2. Verfahren nach Anspruch 1, **dadurch gekennzeichnet, daß** in den Formeln
R¹ für geradkettiges oder verzweigtes C₁-C₄-Alkyl oder gegebenenfalls substituiertes Benzyl steht,
R² und R³ unabhängig voneinander jeweils für Wasserstoff, geradkettiges oder verzweigtes, gegebenenfalls durch Halogen substituiertes C₁-C₄-Alkyl, gegebenenfalls durch Halogen substituiertes C₃-C₆-Cycloalkyl oder gegebenenfalls substituiertes Benzyl stehen und
R⁴ für geradkettiges oder verzweigtes C₁-C₄-Alkyl steht.

3. Verfahren nach Ansprüchen 1 bis 2, **dadurch gekennzeichnet, daß** man das Alkylhydrazin der Formel (III) in äquimolarer Menge oder im Überschuß vorlegt und den Diketoester der Formel (II) langsam zugibt.

4. Verfahren nach Ansprüchen 1 bis 3, **dadurch gekennzeichnet, daß** man eine kleine Menge freies Alkylhydrazin vorlegt und den Rest des Alkylhydrazins und den Diketoester simultan zugibt.

5. Verfahren nach Ansprüchen 1 bis 4, **dadurch gekennzeichnet, daß** man den Diketoester der Formel (II) in Form seines Enolates der Formel 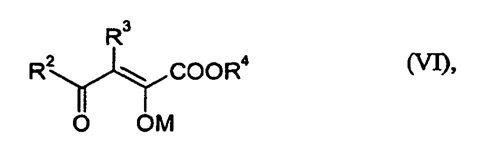 in der
R², R³ und R⁴ die in Anspruch 1 bei Formel (I) angegebene Bedeutung haben
und
M für ein Äquivalent eines Alkali- oder Erdalkalimetalls steht, einsetzt.

6. Verfahren nach Ansprüchen 1 bis 5, **dadurch gekennzeichnet, daß** man als Alkylhydraziniumsalze solche der Formel (VII) einsetzt
[R¹NH₂-NH₂]⁺ X⁻ (VIII),
in der
R¹ die in Anspruch 1 bei Formel (I) angegebene Bedeutung hat und
X⁻ für das Anion einer organischen oder anorganischen Säure steht.

7. Verfahren nach Ansprüchen 1 bis 6, **dadurch gekennzeichnet, daß** man den 2,4-Diketoester in Form seines Enolates einsetzt, wie es als rohe Reaktionsmischung bei der Kondensation zwischen einem Dialkylketon der Formel 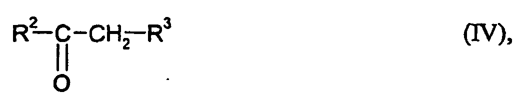 in der
R² und R³ die in Anspruch 1 bei Formel (I) angegebenen Bedeutungen haben,
mit einem Oxalester der Formel
R⁴OOC-COOR⁴ (V),
in der
R⁴ die in Anspruch 1 bei Formel (I) angegebene Bedeutung hat,
anfällt und diesen zu einem Gemisch eines Alkylhydraziniumsalzes der Formel
[R¹NH₂-NH₂]⁺ X⁻ (VII),
in der
R¹ die in Anspruch 1 bei Formel (I) angegebene Bedeutung hat und
X⁻ für das Anion einer organischen oder anorganischen Säure steht,
und einen Anteil an freiem Alkylhydrazin der Formel (III) enthält, zudosiert.

8. Verfahren nach Ansprüchen 1 bis 7, **dadurch gekennzeichnet, daß** man es bei Temperaturen zwischen -20 und +100°C und Reaktionszeiten (= Zeit für das Zusammengeben der Reaktionspartner + Nachrührzeit) zwischen 0,5 und 12 Stunden durchführt.

## Claims

1. Process for preparing 1-alkyl-pyrazole-5-carboxylic esters of the formula 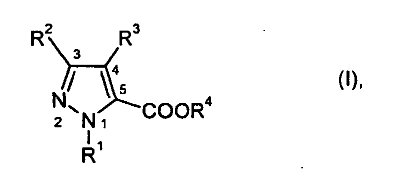 in which
R¹ and R⁴ independently of one another each represent straight-chain or branched, optionally halogen-substituted C₁-C₆-alkyl, optionally halogen-substituted C₃-C₇-cycloalkyl or optionally substituted C₇-C₁₂-aralkyl and
R² and R³ independently of one another each represent hydrogen, straight-chain or branched C₁-C₆-alkyl, C₃-C₇-cycloalkyl or optionally substituted C₇-C₁₂-aralkyl,
**characterized in that** a 2,4-diketo ester of the formula 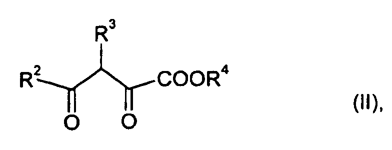 in which
R², R³ and R⁴ are each as defined in formula (I),
and/or an enolate thereof is added
to an alkylhydrazine of the formula
R¹-NH-NH (III),
in which
R¹ is as defined in formula (I),
and/or a corresponding alkylhydrazinium salt,
if appropriate in the presence of a solvent and/or of water, and furthermore such an amount of free alkylhydrazine of the formula (III) and such an amount of alkylhydrazinium salt are used that, after the reaction has ended, from 5 to 200 mol% of free alkylhydrazine of the formula (III) are still present (based on the diketo ester employed or its enolate).

2. Process according to Claim 1, **characterized in that** in the formulae
R¹ represents straight-chain or branched C₁-C₄-alkyl or optionally substituted benzyl,
R² and R³ independently of one another each represent hydrogen, straight-chain or branched, optionally halogen-substituted C₁-C₄-alkyl, optionally halogen-substituted C₃-C₆-cycloalkyl or optionally substituted benzyl and
R⁴ represents straight-chain or branched C₁-C₄-alkyl.

3. Process according to Claims 1 to 2, **characterized in that** the alkylhydrazine of the formula (III) is initially charged in an equimolar amount or in excess and the diketo ester of the formula (II) is slowly added.

4. Process according to Claims 1 to 3, **characterized in that** a small amount of free alkylhydrazine is initially charged and the remainder of the alkylhydrazine and the diketo ester are added simultaneously.

5. Process according to Claims 1 to 4, **characterized in that** the diketo ester of the formula (II) is employed in the form of its enolate of the formula 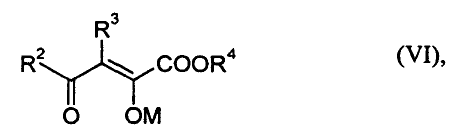 in which
R², R³ and R⁴ are each as defined in Claim 1 for formula (I)
and
M represents an equivalent of an alkali metal or alkaline earth metal.

6. Process according to Claims 1 to 5, **characterized in that** the alkylhydrazinium salts employed are those of the formula (VII)
[R¹NH₂-NH₂]⁺X⁻ (VII),
in which
R¹ is as defined in Claim 1 for formula (I) and
X⁻ represents the anion of an organic or inorganic acid.

7. Process according to Claims 1 to 6, **characterized in that** the 2,4-diketo ester is employed in the form of its enolate as obtained as a crude reaction mixture in the condensation of a dialkyl ketone of the formula 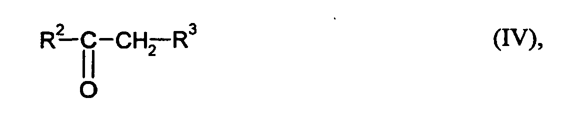 in which
R² and R³ are each as defined in Claim 1 for formula (I)
with an oxalic ester of the formula
R⁴OOC-COOR⁴ (V),
in which
R⁴ is as defined in Claim 1 for formula (I),
and this is metered into a mixture of an alkylhydrazinium salt of the formula
[R¹NH₂-NH₂]⁺ X⁻ (VII),
in which
R¹ is as defined in Claim 1 for formula (I) and
X⁻ represents the anion of an organic or inorganic acid,
and which contains a proportion of free alkylhydrazine of the formula (III).

8. Process according to Claims 1 to 7, **characterized in that** the process is carried out at temperatures between -20 and +100°C and with reaction times (= time for mixing the reaction partners + subsequent stirring time) between 0.5 and 12 hours.

## Revendications

1. Procédé de fabrication d'esters d'acides 1-alkyl-pyrazole-5-carboxyliques de la formule 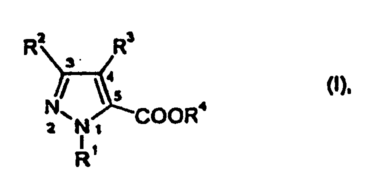 dans laquelle
R¹ et R⁴ représentent indépendamment l'un de l'autre respectivement un alkyle en C₁-C₆ en chaîne linéaire ou ramifié, le cas échéant substitué par un halogène, un cycloalkyle en C₃-C₇, le cas échéant substitué par un halogène, ou un aralkyle en C₁-C₁₂, le cas échéant substitué, et
R² et R³ représentent indépendamment l'un de l'autre respectivement l'hydrogène, un alkyle en C₁-C₆ en chaîne linéaire ou ramifié, le cas échéant substitué par un halogène, un cycloalkyle en C₃-C₇ ou un aralkyle en C₇-C₁₂, le cas échéant substitué,
**caractérisé en ce qu'**on fait réagir un ester 2,4-dicétonique de la formule 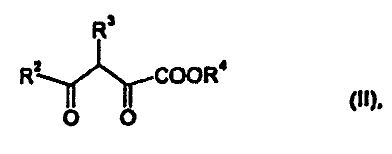 dans laquelle
R², R³ et R⁴ ont la signification indiquée pour la formule (I),
et/ou un énolate de celui-ci
avec une alkylhydrazine de la formule
R¹-NH-NH₂ (III),
dans laquelle
R¹ a la signification indiquée pour la formule (I),
et/ou un sel correspondant d'alkylhydrazinium,
le cas échéant en présence d'un solvant et/ou d'eau, et **en ce qu'**on utilise en outre suffisamment d'alkylhydrazine libre de la formule (III) et suffisamment de sel d'alkylhydrazinium pour se trouver à la fin de la conversion en présence d'encore 5 à 200 % en moles d'alkylhydrazine libre de la formule (III) (rapportés à l'ester dicétonique utilisé ou à son énolate).

2. Procédé suivant la revendication 1, **caractérisé en ce que**, dans les formules,
R¹ représente un alkyle en C₁-C₄ en chaîne linéaire ou ramifié ou un benzyle, le cas échéant substitué,
R² et R³ représentent indépendamment l'un de l'autre respectivement l'hydrogène, un alkyle en C₁-C₄ en chaîne linéaire ou ramifié, le cas échéant substitué par un halogène, un cycloalkyle en C₃-C₆, le cas échéant substitué par un halogène, ou un benzyle, le cas échéant substitué, et
R⁴ représente un alkyle en C₁-C₄ en chaîne linéaire ou ramifié.

3. Procédé suivant les revendications 1 à 2, **caractérisé en ce qu'**on introduit l'alkylhydrazine de la formule (III) en quantité équimolaire ou en excès et qu'on ajoute lentement l'ester dicétonique de la formule (II).

4. Procédé suivant les revendications 1 à 3, **caractérisé en ce qu'**on introduit une petite quantité d'alkylhydrazine libre et qu'on ajoute simultanément le reste de l'alkylhydrazine et l'ester dicétonique.

5. Procédé suivant les revendications 1 à 4, **caractérisé en ce qu'**on utilise l'ester dicétonique de la formule (II) sous la forme de son énolate de la formule 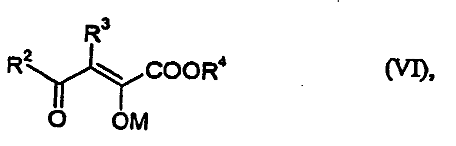 dans laquelle
R², R³ et R⁴ ont la signification indiquée dans la revendication 1 pour la formule (I) et
M représente un équivalent d'un métal alcalin ou alcalino-terreux.

6. Procédé suivant les revendications 1 à 5, **caractérisé en ce qu'**on utilise comme sels d'alkylhydrazinium ceux de la formule (VII)
[R¹NH₂-NH₂]⁺ X⁻ (VII),
dans laquelle
R¹ a la signification indiquée dans la revendication 1 pour la formule (I) et
X⁻ représente l'anion d'un acide organique ou inorganique.

7. Procédé suivant les revendications 1 à 6, **caractérisé en ce qu'**on utilise l'ester 2,4-dicétonique sous la forme de son énolate, tel qu'on l'obtient comme mélange brut de réaction lors de la condensation d'une dialkylcétone de la formule
dans laquelle
R² et R³ ont la signification indiquée dans la revendication 1 pour la formule (I),
avec un ester oxalique de la formule
R⁴OOC-COOR⁴ (V),
dans laquelle
R⁴ a la signification indiquée dans la revendication 1 pour la formule (I),
et qu'on ajoute celui-ci à un mélange d'un sel d'alkylhydrazinium de la formule
[R¹NH₂-NH₂]⁺ X⁻ (VII),
dans laquelle
R¹ a la signification indiquée dans la revendication 1 pour la formule (I) et
X⁻ représente l'anion d'un acide organique ou inorganique,
et qui contient une partie aliquote d'alkylhydrazine libre de la formule (III).

8. Procédé suivant les revendications 1 à 7, **caractérisé en ce qu'**on l'effectue à des températures entre -20 et +100°C avec des temps de réaction (= temps pour l'addition des agents réactifs + temps de mélange subséquent) compris entre 0,5 et 12 heures.
